# EUROPEAN PATENT APPLICATION

(11) **EP 1 908 756 A1**
(43) Date of publication of application: **09.04.2008**
(21) Application number: 07023241.8
(22) Date of filing: 20.07.2005
(51) Int. Cl.: C07D 307/88

(54) **Processes for preparation of crystalline mycophenolate sodium**

(30) Priority: 20.07.2004 US 589909 P; 29.11.2004 US 631849 P
(62) Divisional of application: 05775059.8
(71) Applicant: TEVA Gyógyszergyár Zártkörüen Müködö Részvénytársaság, Pallagi ut 13 4042 Debrecen (HU)
(72) Inventor: Molnar, Sandor, 4031 Debrecen (HU); Szabo, Csaba, 4031 Debrecen (HU); Kovacsne-Mezei, Adrienne, 4032 Debrecen (HU); Tamas, Tivadar, 4034 Debrecen (HU); Hajko, Janos, 4028 Debrecen (HU); Aronhime, Judith, 76217 Rehovot (IL)
(74) Representative: Wong, Kit Yee

(57) **Abstract**

Provided are crystalline mycophenolate sodium forms and processes for their preparation.

## Description

### CROSS-REFERENCE TO RELATED APPLICATIONS

This application claims the benefits of U.S. Provisional Patent Application Nos. 60/589,909, filed July 20, 2004, and 60/631,849 filed November 29, 2004, the contents of all of which are incorporated herein by reference.

### FIELD OF THE INVENTION

The present invention relates to the solid state chemistry of mycophenolate sodium.

### BACKGROUND OF THE INVENTION

At the end of 1960's, Eli Lilly disclosed the inhibiting effect of mycophenolate sodium salt (MPS) on the growth of malignant tumor cells in warm-blooded mammals. Nowadays Novartis has introduced an enteric-coated formulation of mycophenolate sodium, referred to as Myfortic^{®}. Mycophenolic acid can be formed either as mono- or disodium salt. South African patent No. 6804959 describes the preparation of mono- and disodium mycophenolate. Monosodium mycophenolate can be isolated after reaction of one molar equivalent of sodium methoxide with mycophenolic acid in a mixture of methanol and chloroform by precipitation with *n*-pentane. Preparation of the corresponding disodium salt is also described. In this case two molar equivalents of sodium methoxide were added to a solution of mycophenolic acid in 2:1 benzene-chlorofonn mixture. The evaporated material was crystallized from aqueous acetone.

The synthetic route of WO 97/38689 is identical to the one described in South African patent No. 6804959. The compound may be obtained in crystalline form by recrystallization from acetone/ethanol if necessary with water (m.p.189-191°C).

The present invention relates to the solid state physical properties of mycophenolate sodium. These properties may be influenced by controlling the conditions under which mycophenolate sodium is obtained in solid form. Solid state physical properties include, for example, the flowability of the milled solid. Flowability affects the ease with which the material is handled during processing into a pharmaceutical product. When particles of the powdered compound do not flow past each other easily, a formulation specialist must take that fact into account in developing a tablet or capsule formulation, which may necessitate the use of glidants such as colloidal silicon dioxide, talc, starch or tribasic calcium phosphate.

Another important solid state property of a pharmaceutical compound is its rate of dissolution in aqueous fluid. The rate of dissolution of an active ingredient in a patient's stomach fluid may have therapeutic consequences since it imposes an upper limit on the rate at which an orally-administered active ingredient may reach the patient's bloodstream. The rate of dissolution is also a consideration in formulating syrups, elixirs and other liquid medicaments. The solid state form of a compound may also affect its behavior on compaction and its storage stability.

These practical physical characteristics are influenced by the conformation and orientation of molecules in the unit cell, which defines a particular polymorphic Form of a substance. The polymorphic form may give rise to thermal behavior different from that of the amorphous material or another polymorphic form. Thermal behavior is measured in the laboratory by such techniques as capillary melting point, thermogravimetric analysis (TGA) and differential scanning calorimetry (DSC) and may be used to distinguish some polymorphic forms from others. A particular polymorphic form may also give rise to distinct spectroscopic properties that may be detectable by powder X-ray crystallography, solid state C NMR spectrometry and infrared spectrometry.

W02004/020426 discloses preparation of sodium mycophenolate by reacting mycophenolic acid or its ammonium or dibenzyl-amine salt with a sodium salt of C₂ to C₁₀ carboxylic acid. Mycophenolic acid is converted to its ammonium salt by reacting with ammonia. This compound is reacted with sodium acetate to obtain the sodium salt of mycophenolic acid.

WO 2004/064806 discloses additional polymorphic forms of mycophenolate sodium and acid.

### Monosodium Salt

South African patent No. 68/4,959 provides an example for preparing monosodium mycophenolate salt (Example 3). Sodium methylate in anhydrous methanol was added to mycophenolic acid in chloroform, then the monosodium salt was precipitated by adding n-pentane and collected by filtration and vacuum dried.

Acta Chrystallographica Sect. C, (2000), C56, 432-433 describes another process for producing monosodium mycophenolate. A methanolic solution of the commercially available mycophenolic acid was treated with one equivalent of sodium methanolate. After stirring for 1 hour at room temperature, the solvent was evaporated to dryness in vacuum. The melting point of the product was 463 K (190°C). Single crystals were grown by evaporation and cooling of a water/ethyl acetate solution from about 323K to room temperature. The crystal structure of the produced sodium mycophenolate measured by single crystal diffractometer is also described.

Based on the given crystal parameters, the calculated powder diffractogram done by the inventors of the present invention show that the crystal form obtained is the crystal form denominated Form M2. Form M2 is an anhydrous form. Form M2 is characterized by a powder XRD pattern with peaks at 5.3, 8.0, 9.8, 10.7, and 21.9 ±0.2 degrees 2 theta (Fig. 3) and FTIR peaks at 1719, 1571, 1317, 1266, 1134 and 927 cm-1 (Fig. 4). Form M2 may be further characterized by XRD peaks at 13.6 and 19.0 ±0.2 degrees 2 theta. Form M2 may be further characterized by IR peaks at 1194, 1108, 1075, 1034, 971, 875, 826, 794 and 722 cm-1. Form M2 may be further characterized by a DSC curve (Fig. 43).

PCT 97/38689 describes sodium mycophenolate salts as known from South African Patent. It also describes the process for obtaining monosodium salt in crystalline form by recrystallization from acetone/ethanol if necessary with water. The melting point provided is 189-191 °C.

J. Med. Chem. (1996), 39, 1236-1242 describes treating a solution of mycophenolic acid in ethanol with equimolar sodium ethylate at room temperature and stirring for 30 minutes. The solvent was evaporated in vacuum.

J. Pharm. Sciences (1970), 59(8), 1157-1159 asserts that monosodium mycophenolate may be formed by adjusting the slurry of mycophenolic acid to pH 7-8 with sodium hydroxide. No physical data is provided.

South African patent No. 68/4,959 provides an example for producing disodium mycophenolate (Example 2). Mycophenolic acid was dissolved in benzene:chloroform 2:1 solvent mixture and sodium methoxide dissolved in anhydrous methanol was added to it. The solution was stirred for 15-20 minutes, evaporated to dryness and redissolved in water. Crystallization was effected by the addition of acetone to the hot water solution and chilling overnight. No physical data was given.

The discovery of new polymorphic forms of a pharmaceutically useful compound and/or new processes for their preparation provides a new opportunity to improve the performance characteristics of a pharmaceutical product. It enlarges the repertoire of materials that a formulation scientist has available for designing, for example, a pharmaceutical dosage form of a drug with a targeted release profile or other desired characteristic.

### SUMMARY OF THE INVENTION

In one embodiment, the present invention provides a process for preparing anhydrous crystalline mycophenolate sodium (Form M2) characterized by a powder XRD pattern with peaks at 5.3, 8.0, 9.8, 10.7, and 21.9 ± 0.2 degrees 2-theta comprising the steps of:
(a) preparing a solution of mycophenolic acid in a C1-C4 alcohol;
(b) combining a base and a source of sodium with the solution to obtain a reaction mixture;
(c) crystallizing the crystalline form the mixture; and
(d) recovering the crystalline form.

In another embodiment, the present invention provides a process for preparing anhydrous crystalline mycophenolate sodium (Form M2) characterized by a powder XRD pattern with peaks at 5.3, 8.0, 9.8, 10.7, and 21.9 ± 0.2 degrees 2-theta comprising the steps of:
(a) preparing a solution of mycophenolic acid in a C3-C8 ester,
(b) combining a base and a source of sodium with the solution to precipitate the crystalline form;
(c) recovering the crystalline form.

In another embodiment, the present invention provides a process for preparing anhydrous crystalline mycophenolate sodium (Form M2) characterized by a powder XRD pattern with peaks at 5.3, 8.0, 9.8, 10.7, and 21.9 ± 0.2 degrees 2-theta comprising the steps of:
(a) preparing a solution of sodium mycophenolate in 1-butanol;
(b) crystallizing the crystalline form from the solution; and
(c) recovering the crystalline form.

In another embodiment, the present invention provides a process for preparing anhydrous crystalline mycophenolate sodium (Form M2) characterized by a powder XRD pattern with peaks at 5.3, 8.0, 9.8, 10.7, and 21.9 ± 0.2 degrees 2-theta comprising the steps of:
(a) preparing a solution of mycophenolic acid in a C3-C8 ketone;
(b) combining a base and a source of sodium with the solution to precipitate the crystalline form; and
(c) recovering the crystalline form.

In another embodiment, the present invention provides a process for preparing anhydrous crystalline mycophenolate sodium (Form M2) characterized by a powder XRD pattern with peaks at 5.3, 8.0, 9.8, 10.7, and 21.9 ± 0.2 degrees 2-theta comprising the steps of:
(a) preparing a solution of sodium mycophenolate in ethanol;
(b) crystallizing the crystalline form from the solution;
(c) recovering the crystalline form; and
(d) drying the recovered crystalline form.

In another embodiment, the present invention provides a process for preparing anhydrous crystalline mycophenolate sodium (Form M2) characterized by a powder XRD pattern with peaks at 5.3, 8.0, 9.8, 10.7, and 21.9 ± 0.2 degrees 2-theta comprising the steps of:
(a) preparing a solution of sodium mycophenolate in ethyl lactate;
(b) combining acetone with the solution to precipitate the crystalline form; and
(c) recovering the crystalline form.

In another embodiment, the present invention provides a process for preparing anhydrous crystalline mycophenolate sodium (Form M2) characterized by a powder XRD pattern with peaks at 5.3, 8.0, 9.8, 10.7, and 21.9 ± 0.2 degrees 2-theta comprising the steps of:
(a) preparing a solution of sodium mycophenolate in dimethylformide;
(b) combining 1-propanol with the solution to precipitate the crystalline form;
(c) recovering the crystalline form; and
(d) drying the recovered crystalline form.

In another embodiment, the present invention provides a process for preparing anhydrous crystalline mycophenolate sodium (Form M2) characterized by a powder XRD pattern with peaks at 5.3, 8.0, 9.8, 10.7, and 21.9 ± 0.2 degrees 2-theta comprising the steps of:
(a) preparing a solution of sodium mycophenolate in methanol;
(b) combining an antisolvent with the solution to precipitate the crystalline form;
(c) recovering the crystalline form; and
(d) drying the recovered crystalline form,
wherein the antisolvent is selected from the group consisting of ethyl acetate, methyl-tert-butyl ether, and mixtures thereof.

In another embodiment, the present invention provides a process for preparing anhydrous crystalline mycophenolate sodium (Form M2) characterized by a powder XRD pattern with peaks at 5.3, 8.0, 9.8, 10.7, and 21.9 ± 0.2 degrees 2-theta comprising the steps of:
(a) preparing a solution of sodium mycophenolate in methanol;
(b) combining the solution with an antisolvent to precipitate the crystalline form; and
(c) recovering the crystalline form,
wherein the antisolvent solvent is selected from the group consisting of acetone, isopropanol, tetrahydrofuran, diisopropyl ether, nitromethane, isobutanol, and mixtures thereof.

In another embodiment, the present invention provides a process for preparing anhydrous crystalline mycophenolate sodium (Form M2) characterized by a powder XRD pattern with peaks at 5.3, 8.0, 9.8, 10.7, and 21.9 ± 0.2 degrees 2-theta comprising the steps of:
(a) preparing a solution of mycophenolic acid in a mixture of dichloromethane and methanol; and
(b) combining a base and a source of sodium with the solution to precipitate the crystalline form; and
(c) recovering the crystalline form.

In another embodiment, the present invention provides a process for preparing anhydrous crystalline mycophenolate sodium (Form M2) characterized by a powder XRD pattern with peaks at 5.3, 8.0, 9.8, 10.7, and 21.9 ± 0.2 degrees 2-theta comprising the steps of heating a solid mixture of a crystalline mycophenolate sodium (Form M1) characterized by a powder XRD pattern with peaks at 4.7, 6.6, 11.2 and 15.6 ± 0.2 degrees 2-theta and a crystalline mycophenolate sodium (Form M3) characterized by at least one of a powder XRD pattern with peaks at 6.0, 9.3, 15.5, and 18.4 ± 0.2 degrees 2-theta.

### BRIEF DESCRIPTION OF THE FIGURES

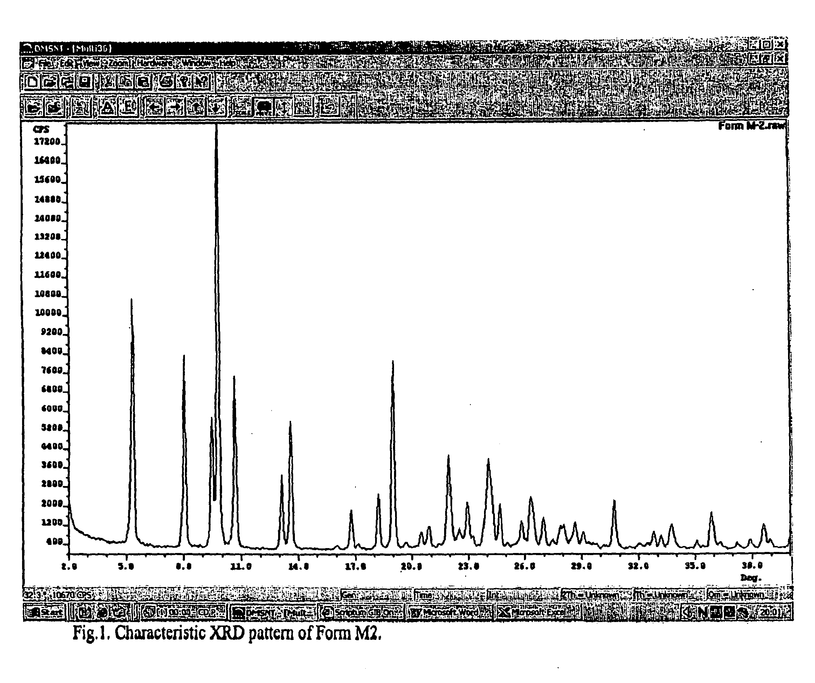

Figure 1 is a characteristic X-ray powder diffraction pattern for monosodium mycophenolate form M2.

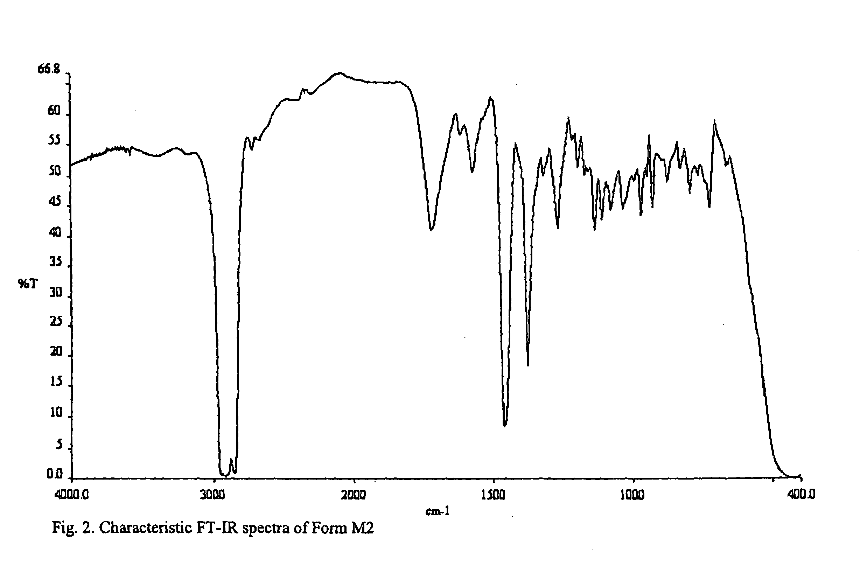

Figure 2 is a characteristic FT-IR spectrum of monosodium mycophenolate form M2.

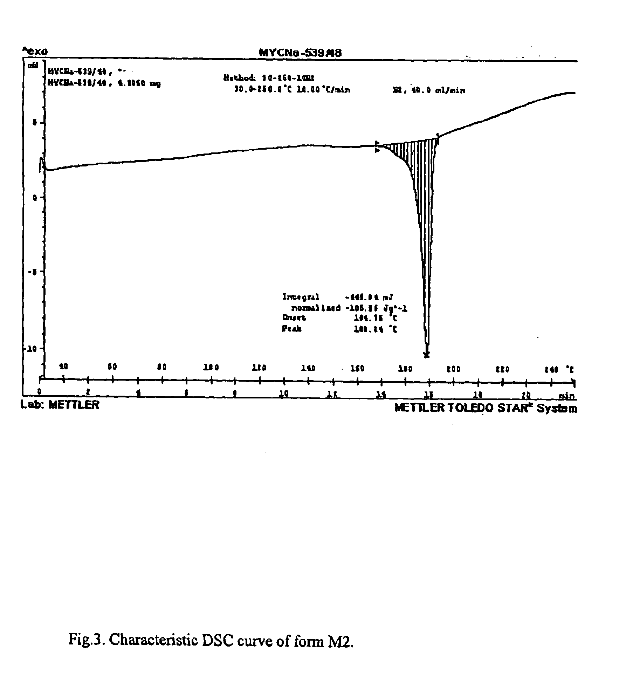

Figure 3 is a characteristic DSC curve for monosodium mycophenolate form M2.

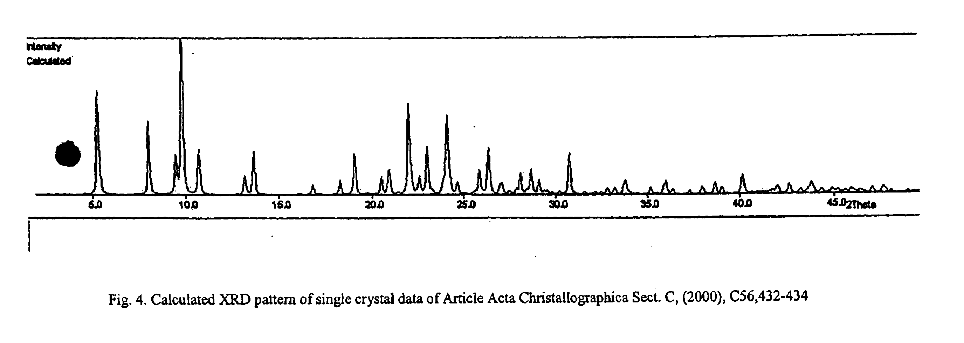

Figure 4 is a calculated XRD pattern of a single crystal data of article Acta Crystallographica Sect. C, (2000), C56, 432-434.

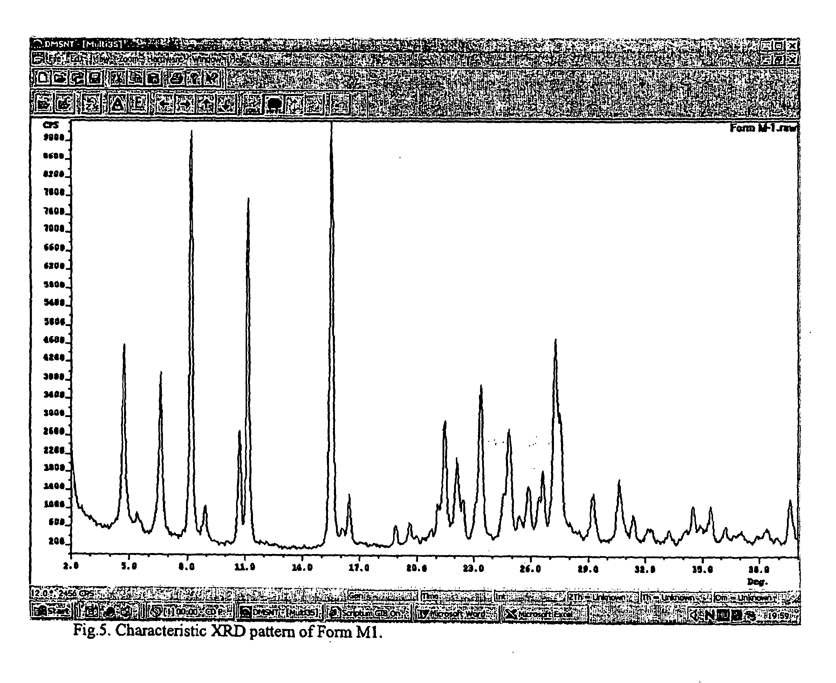

Figure 5 is a characteristic X-ray powder diffraction pattern for monosodium mycophenolate form M 1.

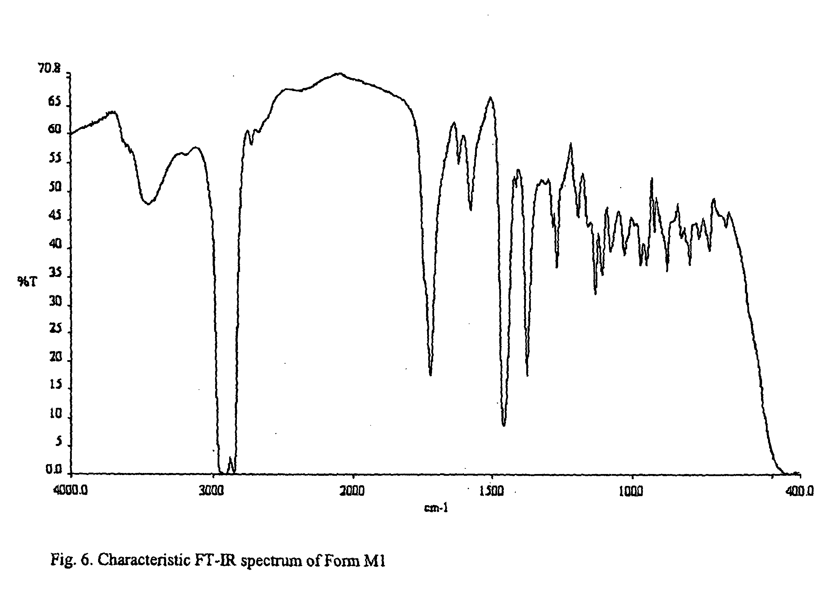

Figure 6 is a characteristic FT-IR spectrum of monosodium mycophenolate form M1.

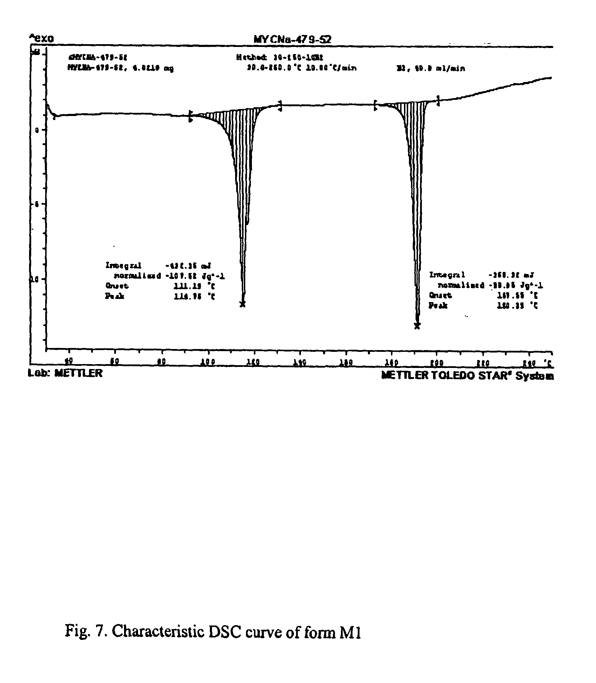

Figure 7 is a characteristic DSC curve for monosodium mycophenolate form M1.

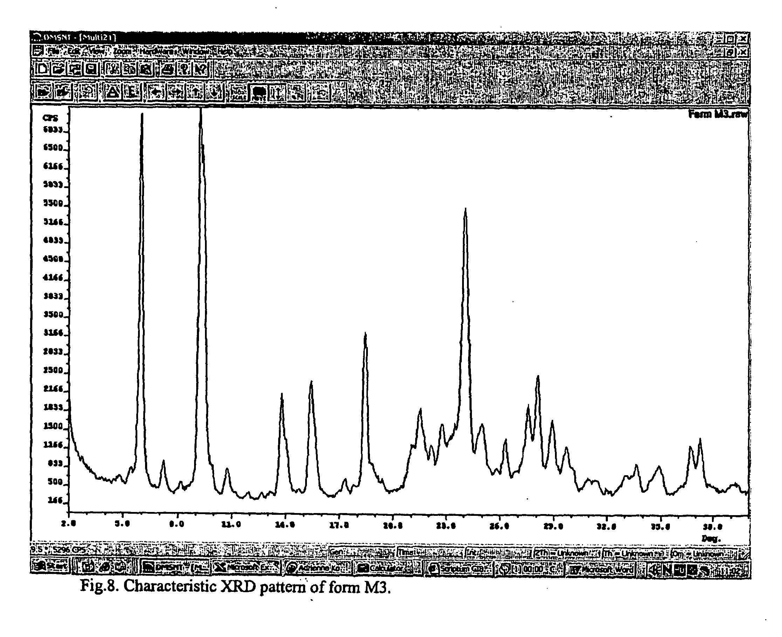

Figure 8 is a characteristic X-ray powder diffraction pattern for monosodium mycophenolate form M3.

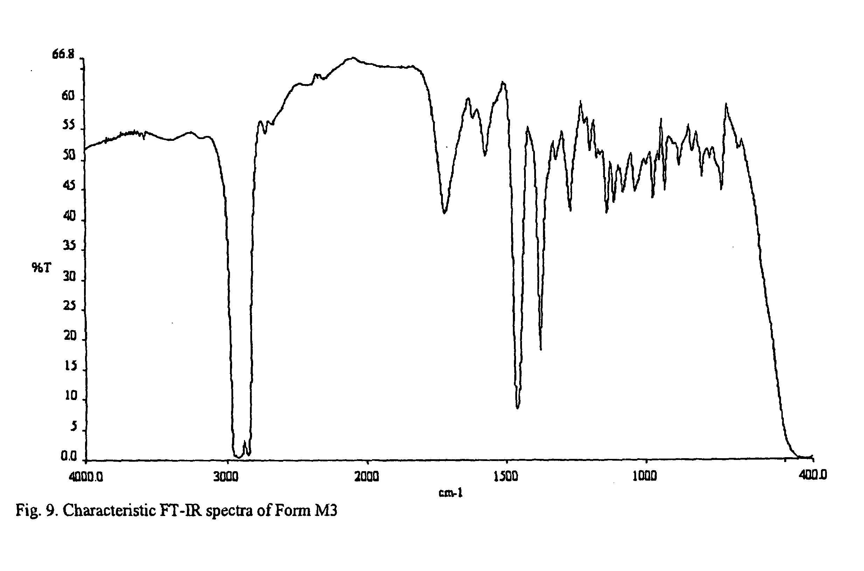

Figure 9 is a characteristic FT-IR spectrum of monosodium mycophenolate form M3.

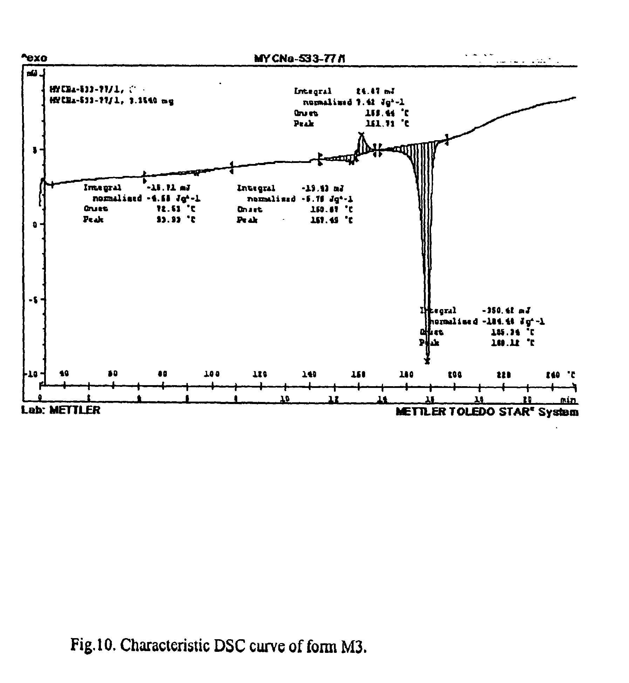

Figure 10 is a characteristic DSC curve for monosodium mycophenolate form M3.

### DETAILED DESCRIPTION OF THE INVENTION

The present invention provides various processes for preparation of Form M2 of mycophenolate sodium on an industrial scale. Most of the processes developed in the present invention are crystallization or slurry processes, which are suitable for use on an industrial scale (batches of at least 0.5Kg). Further, crystallization often allows for obtaining a product with higher purity.

Form M2 may be prepared by slurry or crystallization from a solution of the sodium salt in a C₃ to C₈ organic ester as solvent, such as ethyl acetate or isobutyl acetate. The solution or slurry may contain charcoal for removal of colored impurities, which charcoal is then removed by filtration. To prepare a solution of the sodium salt, a source of sodium is added to the solution of MPA in the ester. In one embodiment, a base in methanol, such as about a 30% solution of sodium methoxide in methanol is added portion-wise, preferably at a temperature of about 10 to about 60°C, most preferably at about room temperature. The reaction mixture is preferably stirred for an additional approximately of 1 or 2 hours. A solid precipitates from the solution, which is then recovered by conventional techniques. The solid material is then preferably dried, preferably at about 40 to about 85°C in a vacuum oven. A slurry process with such ester is illustrated in example 3.

Form M2 may be prepared by crystallization from a C₁-C₄ alcohol, preferably methanol. In this embodiment, a solution of the mycophenolic acid in the alcohol is combined with a base or source of sodium, preferably sodium methoxide. The combining may be carried out portion-wise, or in one step. The resulting reaction mixture may be heated to a temperature of at least about 35°C to obtain complete dissolution, though the reaction may be carried out without heating. A crystalline form is then crystallized from the solution, preferably by cooling, more preferably by cooling to a temperature of less than about 0°C, most preferably about -10°C to about -20°C. The crystals are then recovered and may be dried. Analysis of the dried product showed that it was Form M2. During this process, the reaction mixture is kept for at least about 5 hours during crystallization.
In other embodiments, an antisolvent is added to the methanol to precipitate Form M2. In this embodiment, sodium mycophenolate is dissolved in methanol, preferably at room temperature, then a halogen-free solvent such as a C₃ to C₈ ketone (preferably acetone) or ester (preferably ethyl acetate iso-butyl acetate) or a C₂ to C₈ ether (preferably tetrahydrofuran or t-butyl methyl ether or diisopropyl ether) or a C₂ to C₄ alcohol (preferably isopropanol or isobutanol) is added to the solution. Nitromethane may also be used. A solid then crystallizes from the solution, preferably at room temperature overnight. The solid is then recovered, and preferably dried at room temperature. With ethyl acetate and methyl-t-butyl-ether as anti-solvents, the product may have to be dried to obtain Form M2. Drying may be carried out at room temperature and/or under atmospheric pressure.

Form M2 of sodium mycophenolate may also be obtained when sodium mycophenolate (MPS) is crystallized from ethanol or 1-butanol. The ethanol is preferably absolute ethanol. In one embodiment, sodium mycophenolate is dissolved in ethanol or 1-butanol at ambient or elevated temperature, cooled to room temperature and crystallized at this temperature for overnight. The solution may be heated to a temperature of at least about, 60°C, such as the reflux temperature of the solvent. The crystals obtained from ethanol may have to be dried to obtain Form M2. Drying may be carried out at room temperature under atmospheric pressure (ambient conditions).

In another embodiment, mycophenolate sodium Form M2 is prepared by precipitating Form M2 by adding acetone as an antisolvent to a solution of mycophenolate sodium in ethyl lactate. In one embodiment, sodium mycophenolate is dissolved in ethyl lactate at elevated temperature, such as about 60°C. The solution is then cooled, preferably to about room temperature, and acetone is added to the solution. After addition of the acetone, the solution is further cooled, preferably to about 0°C to about 10°C and allowed to crystallize. The product is then recovered by conventional techniques and may be dried.

In another embodiment, mycophenolate sodium Form M2 is prepared by precipitating Form M2 by adding 2-propanol as an antisolvent to a solution of mycophenolate sodium in dimethylformide. As exemplified, in this embodiment, 2-propanol is combined with a solution of sodium mycophenolate in dimethylformide and allowed to crystallize. The product is then recovered by conventional techniques. The product may have to be dried to obtain Form M2. The crystalline form may be dried at room temperature and/or under atmospheric pressure.

Form M2 may also be obtained by crystallization from a C₃ to C₈ ketone, such as acetone. In this embodiment, a base and a source of sodium, preferably an aqueous solution of sodium hydroxide, is added to a solution of MPA in acetone. Examples of other bases include sodium methoxide and sodium ethoxide. The reaction is preferably carried out with stirring. The solid product is then recovered by conventional techniques and may be dried.

Another process for preparing crystalline mycophenolate sodium M2 comprises heating a solid mixture of crystalline mycophenolate sodium Form M1 and Form M3. In a preferred embodiment, mycophenolate sodium Form M2 is prepared by heating a solid mixture of mycophenolate sodium Forms M1 and M3 in an oven at about 170°C to obtain Form M2, preferably about 30 minutes, and placing the mixture in a desicator to allow it to cool to room temperature.

In another embodiment, mycophenolate sodium Form M2 by adding a base and a source of sodium to a solution of mycophenolic acid in a mixture of dichloromethane and methanol. Preferably sodium methoxide in methanol is added to the solution. To further induce precipitation, a C₅ to C₇ cyclic or acyclic saturated hydrocarbon, such as n-hexane, may be added.

The hygroscopicity of monosodium mycophenolate crystal Form M2 was investigated. Form M2 was exposed to different level of humidity for one week and after equilibrium it was analysed by TGA and XRD for water content and crystal structure. Table 5 summarizes the results:

**Table 5.**

| **Crystal forms** | **Results** | | |
|---|---|---|---|
| | **%RH** | **LOD(%)** | **Form** |
| M2 (example 3) | 40 | 0.1 | M2 |
| | 60 | 0.2 | M2 |
| | 80 | 0.4 | M2 |

Crystalline Form M2 obtained with the processes of the present invention preferably contains less than 30%, more preferably less than 20%, more preferably less than 10%, and most preferably less than about 5% of other polymorphic forms by weight.

The single particle size for Form M2 is less than about 100 micrometers, as measured by polarizing light microscope of crystal described in the invention.

The starting material used for the processes of the present invention, unless otherwise specified, may be any crystalline or amorphous form of mycophenolate sodium or acid, including various solvates and hydrates.

The processes of the present invention may also be practiced as the last step of prior art processes that synthesize mycophenolate sodium.

The base and the source of sodium as used throughout this invention can be different, or they can be the same. For example, sodium methoxide, sodium ethoxide, or sodium hydroxide can be used as both the base and the source of sodium. The preferred base and source of sodium is sodium methoxide.

Many processes of the present invention involve crystallization out of a particular solvent. One skilled in the art would appreciate that the conditions concerning crystallization may be modified without affecting the form of the polymorph obtained. For example, when mixing mycophenolate sodium in a solvent to form a solution, warming of the mixture may be necessary to completely dissolve the starting material. If warming does not clarify the mixture, the mixture may be diluted or filtered. To filter, the hot mixture may be passed through paper, glass fiber or other membrane material, or a clarifying agent such as celite. Depending upon the equipment used and the concentration and temperature of the solution, the filtration apparatus may need to be preheated to avoid premature crystallization.

The conditions may also be changed to induce precipitation. A preferred way of inducing precipitation is to reduce the solubility of the solvent. The solubility of the solvent may be reduced, for example, by cooling the solvent or adding an anti-solvent.

In one embodiment, an anti-solvent is added to a solution to decrease its solubility for a particular compound, thus resulting in precipitation. Another way of accelerating crystallization is by seeding with a crystal of the product or scratching the inner surface of the crystallization vessel with a glass rod.

Pharmaceutical compositions of the present invention contain mycophenolate sodium Form M2. In addition to the active ingredient(s), the pharmaceutical compositions of the present invention may contain one or more excipients. Excipients are added to the composition for a variety of purposes.

Diluents increase the bulk of a solid pharmaceutical composition and may make a pharmaceutical dosage form containing the composition easier for the patient and care giver to handle. Diluents for solid compositions include, for example, microcrystalline cellulose (e.g. Avicel^{®}), microfine cellulose, lactose, starch, pregelitinized starch, calcium carbonate, calcium sulfate, sugar, dextrates, dextrin, dextrose, dibasic calcium phosphate dihydrate, tribasic calcium phosphate, kaolin, magnesium carbonate, magnesium oxide, maltodextrin, mannitol, polymethacrylates (e.g. Eudragit^{®}), potassium chloride, powdered cellulose, sodium chloride, sorbitol and talc.

Solid pharmaceutical compositions that are compacted into a dosage Form like a tablet may include excipients whose functions include helping to bind the active ingredient and other excipients together after compression. Binders for solid pharmaceutical compositions include acacia, alginic acid, carbomer (e.g. carbopol), carboxymethylcellulose sodium, dextrin, ethyl cellulose, gelatin, guar gum, hydrogenated vegetable oil, hydroxyethyl cellulose, hydroxypropyl cellulose (e.g. Klucel^{®}), hydroxypropyl methyl cellulose (e.g. Methocel^{®}), liquid glucose, magnesium aluminum silicate, maltodextrin, methylcellulose; polymethacrylates, povidone (e.g. Kollidon^{®}, Plasdone^{®}), pregelatinized starch, sodium alginate and starch.

The dissolution rate of a compacted solid pharmaceutical composition in the patient's stomach may be increased by the addition of a disintegrant to the composition. Disintegrants include alginic acid, carboxymethylcellulose calcium, carboxymethylcellulose sodium (e.g. Ac-Di-Sol^{®}, Primellose^{®}), colloidal silicon dioxide, croscarmellose sodium, crospovidone (e.g. Kollidon^{®}, Polyplasdone^{®}), guar gum, magnesium aluminum silicate, methyl cellulose, microcrystalline cellulose, polacrilin potassium, powdered cellulose, pregelatinized starch, sodium alginate, sodium starch glycolate (e.g. Explotab^{®}) and starch.

Glidants can be added to improve the flowability of non-compacted solid composition and improve the accuracy of dosing. Excipients that may function as glidants include colloidal silicon dixoide, magnesium trisilicate, powdered cellulose, starch, talc and tribasic calcium phosphate.

When a dosage Form such as a tablet is made by compaction of a powdered composition, the composition is subjected to pressure from a punch and dye. Some excipients and active ingredients have a tendency to adhere to the surfaces of the punch and dye, which can cause the product to have pitting and other surface irregularities. A lubricant can be added to the composition to reduce adhesion and ease release of the product Form the dye. Lubricants include magnesium stearate, calcium stearate, glyceryl monostearate, glyceryl palmitostearate, hydrogenated castor oil, hydrogenated vegetable oil, mineral oil, polyethylene glycol, sodium benzoate, sodium lauryl sulfate, sodium stearyl fumarate, stearic acid, talc and zinc stearate.

Flavoring agents and flavor enhancers make the dosage Form more palatable to the patient. Common flavoring agents and flavor enhancers for pharmaceutical products that may be included in the composition of the present invention include maltol, vanillin, ethyl vanillin, menthol, citric acid, fumaric acid, ethyl maltol, and tartaric acid

Solid and liquid compositions may also be dyed using any pharmaceutically acceptable colorant to improve their appearance and/or facilitate patient identification of the product and unit dosage level.

In liquid pharmaceutical compositions of the present invention hydrochloride Forms and any other solid excipients are dissolved or suspended in a liquid carrier such as water, vegetable oil, alcohol, polyethylene glycol, propylene glycol or glycerin.

Liquid pharmaceutical compositions may contain emulsifying agents to disperse uniformly throughout the composition an active ingredient or other excipient that is not soluble in the liquid carrier. Emulsifying agents that may be useful in liquid compositions of the present invention include, for example, gelatin, egg yolk, casein, cholesterol, acacia, tragacanth, chondrus, pectin, methyl cellulose, carbomer, cetostearyl alcohol and cetyl alcohol.

Liquid pharmaceutical compositions of the present invention may also contain a viscosity enhancing agent to improve the mouth-feel of the product and/or coat the lining of the gastrointestinal tract. Such agents include acacia, alginic acid bentonite, carbomer, carboxymethylcellulose calcium or sodium, cetostearyl alcohol, methyl cellulose, ethylcellulose, gelatin guar gum, hydroxyethyl cellulose, hydroxypropyl cellulose, hydroxypropyl methyl cellulose, maltodextrin, polyvinyl alcohol, povidone, propylene carbonate, propylene glycol alginate, sodium alginate, sodium starch glycolate, starch tragacanth and xanthan gum.

Sweetening agents such as sorbitol, saccharin, sodium saccharin, sucrose, aspartame, fructose, mannitol and invert sugar may be added to improve the taste. Preservatives and chelating agents such as alcohol, sodium benzoate, butylated hydroxy toluene, butylated hydroxyanisole and ethylenediamine tetraacetic acid may be added at levels safe for ingestion to improve storage stability.

A liquid composition according to the present invention may also contain a buffer such as guconic acid, lactic acid, citric acid or acetic acid, sodium guconate, sodium lactate, sodium citrate or sodium acetate.

Selection of excipients and the amounts to use may be readily determined by the Formulation scientist based upon experience and consideration of standard procedures and reference works in the field.

The solid compositions of the present invention include powders, granulates, aggregates and compacted compositions. The dosages include dosages suitable for oral, buccal, rectal, parenteral (including subcutaneous, intramuscular, and intravenous), inhalant and ophthalmic administration. Although the most suitable route in any given case will depend on the nature and severity of the condition being treated, the most preferred route of the present invention is oral. The dosages may be conveniently presented in unit dosage Form and prepared by any of the methods well-known in the pharmaceutical arts.

Dosage Forms include solid dosage Forms like tablets, powders, capsules, suppositories, sachets, troches and losenges as well as liquid syrups, suspensions and elixirs.

A dosage Form of the present invention is a capsule containing the composition, preferably a powdered or granulated solid composition of the invention, within either a hard or soft shell. The shell may be made from gelatin and optionally contain a plasticizer such as glycerin and sorbitol, and an opacifying agent or colorant. The active ingredient and excipients may be Formulated into compositions and dosage Forms according to methods known in the art.

A composition for tableting or capsule filing may be prepared by wet granulation. In wet granulation some or all of the active ingredients and excipients in powder Form are blended and then further mixed in the presence of a liquid, typically water, that causes the powders to clump up into granules. The granulate is screened and/or milled, dried and then screened and/or milled to the desired particle size. The granulate may then be tableted or other excipients may be added prior to tableting such as a glidant and or lubricant.

A tableting composition may be prepared conventionally by dry blending. For instance, the blended composition of the actives and excipients may be compacted into a slug or a sheet and then comminuted into compacted granules. The compacted granules may be compressed subsequently into a tablet.

As an alternative to dry granulation, a blended composition may be compressed directly into a compacted dosage Form using direct compression techniques. Direct compression produces a more uniform tablet without granules. Excipients that are particularly well suited to direct compression tableting include microcrystalline cellulose, spray dried lactose, dicalcium phosphate dihydrate and colloidal silica. The proper use of these and other excipients in direct compression tableting is known to those in the art with experience and skill in particular Formulation challenges of direct compression tableting.

A capsule filling of the present invention may comprise any of the aforementioned blends and granulates that were described with reference to tableting, only they are not subjected to a final tableting step.

### Abbreviations

MPA = Mycophenolic acid
NaOMe = Sodium methoxide
DMF = Dimethyl formamide

### Experimental methodology (physical)

### XRD

ARL X-ray powder diffractometer model X'TRA-030, Peltier detector, round standard aluminum sample holder with round zero background quartz plate was used. Scanning parameters: Range: 2-40 deg. 2θ, continuous Scan, Rate: 3 deg./min. The accuracy of peak positions is defined as +/- 0.2 degrees due to experimental differences like instrumentations, sample preparations etc.

### FT-IR Spectroscopy

Perkin-Elmer Spectrum 1000 Spectrometer, at 4 cm⁻¹ resolution with 16 scans, in the range of 4000-400 cm⁻¹ was used. The samples were analyzed in Nujol mull. The spectra were recorded using an empty cell as a background.

### Differential Scanning Calorimetry (DSC)

DSC 822°/700, Mettler Toledo, Sample weight: 3-5 mg.
Heating rate: 10 °C/min., Number of holes of the crucible: 3
In N₂ stream: flow rate = 40 ml/min
Scan range: 30-250°C, 10°C/ minutes heating rate.
The DSC curves of the novel forms of mycophenolate monosodium indicates only endothermic peaks due to dehydration, desolvation and melting.

### Thermal Gravimetric Analysis (TGA)

TGA/SDTA 851°, Mettler Toledo, Sample weight 7-15 mg.
Heating rate: 10 °C/ min., In N₂ stream: flow rate = 50 ml/min
Scan range: 30-250°C.

### Microscope

The particle size of single crystals was observed by a polarizing light Microscope, Type : Zeiss TOPIC-B. Sample preparation was done by dispersing a sample in one drop of mineral oil. The magnification was 200.

### Examples

The following examples are offered for illustrative purposes only, and are not intended to limit the scope of the present invention in any way.

### Preparation of mycophenolate monosodium crystal form M2

### Example 1

To a stirred solution ofMPA (6.4 g) in methanol (32 ml), 30% sodium methoxide in methanol (3.8 ml) was added dropwise at room temperature. The reaction mixture was warmed to reflux temperature, then cooled to -15°C with constant stirring. The reaction mixture was stirred at -15°C for 24 hours. The precipitated product was then filtered off and washed with cold methanol. The solid material was dried at 40-45°C in a vacuum oven. Form M2 of mycophenolate sodium was obtained in 38% yield.

### Example 2

To a stirred solution of MPA (6.4 g) in acetone (130 ml), sodium hydroxide (0.8 g) in water (2 ml) was added dropwise at room temperature. The stirring was continued at this temperature for 0.5 hours. The precipitated product was filtered off and washed with cold acetone. The solid material was dried at 40-45 °C in a vacuum oven. Form M2 of mycophenolate sodium was obtained in 85% yield.

### Example 3

A mixture of MPA (128 g) and charcoal (2.56 g) in ethyl acetate (4750 ml) was stirred for 0.5 h, then the charcoal was filtered off and washed with ethyl acetate (250 ml). 30% sodium methoxide in methanol (75.9 ml) was then added dropwise to the stirred filtrate at room temperature. The mixture was stirred for an additional 30 minutes, then the precipitated product was filtered off and washed with ethyl acetate (250 ml). The solid material was dried at 40-45 °C in a vacuum oven. Form M2 of mycophenolate sodium was obtained in 97% yield.

### Example 4

To a stirred solution of MPA (6.4 g) in ethyl acetate (250 ml), 30% sodium methoxide in methanol (3.8 ml) was added dropwise at room temperature. The reaction mixture was stirred for an additional 30 minutes, then the precipitated product was filtered off and washed with ethyl acetate. The solid material was dried at 40-45 °C in a vacuum oven. Form M2 of mycophenolate sodium was obtained in 94% yield.

### Example 5

To a stirred solution of MPA (128 g) in ethyl acetate (5 L), 30% sodium methoxide in methanol (74 ml) was added dropwise at room temperature. The reaction mixture was stirred for an additional 30 minutes, then the precipitated product was filtered off and washed with ethyl acetate. The solid material was dried at 60-65°C in a vacuum oven. Form M2 of mycophenolate sodium was obtained in 96% yield.

### Example 6

To a stirred solution of MPA (24 g) in ethyl acetate (0.93 L), 30% sodium methoxide in methanol (14 ml) was added dropwise at room temperature. The reaction mixture was stirred for an additional 30 minutes, then the precipitated product was filtered off and washed with ethyl acetate. A part of the solid material was divided into three parts and dried at 40-45 °C, 60-65 °C and 80-85 °C in vacuum oven, respectively. Form M2 of mycophenolate sodium was obtained.

### Example 7

Sodium mycophenolate (1 g) was dissolved at reflux temperature in absolute ethanol (165 ml). The solution was allowed to cool to room temperature, and crystallized at this temperature overnight. The solid was filtered off, and a part of the wet material was dried at normal pressure at room temperature. Form M1 of mycophenolate sodium was obtained from the wet sample. Form M2 of mycophenolate sodium was obtained from the dry sample.

### Example 8

Sodium mycophenolate (1 g) was dissolved at reflux temperature in 1-butanol (175 ml). The solution was allowed to cool to room temperature, and crystallized at this temperature overnight. The solid was filtered off, and a part of the wet material was dried at normal pressure at room temperature. Form M2 of mycophenolate sodium was obtained from both the dry and the wet sample.

### Example 9

Sodium mycophenolate (0.5 g) was dissolved at about 60°C in ethyl lactate (5 ml). The solution was allowed to cool to room temperature, then acetone (200 ml) was added to the solution. The solution was stored at +4°C overnight. The solid was filtered off, and a part of the wet material was dried at normal pressure at room temperature. Form M2 of mycophenolate sodium was obtained from both the dry and the wet sample.

### Example 10

Sodium mycophenolate (1 g) was dissolved at room temperature in methanol (15 ml), then acetone (180 ml) was added to the solution. The solution was allowed to stand at room temperature overnight to promote crystallization of the product. The solid was then filtered off, and a part of the wet material was dried at normal pressure at room temperature. Form M2 of mycophenolate sodium was obtained from both the wet and the dry sample.

### Example 11

Sodium mycophenolate (1 g) was dissolved at room temperature in methanol (15 ml), then isopropanol (285 ml) was added to the solution. The solution was allowed to stand at room temperature overnight to promote crystallization of the product. The solid was then filtered off, and a part of the wet material was dried at normal pressure at room temperature. Form M2 of mycophenolate sodium was obtained from both the wet and the dry sample.

### Example 12

Sodium mycophenolate (1 g) was dissolved at room temperature in methanol (15 ml), then tetrahydrofuran (200 ml) was added to the solution. The solution was allowed to stand at room temperature overnight to promote crystallization of the product. The solid was then filtered off, and a part of the wet material was dried at normal pressure at room temperature. Form M2 of mycophenolate sodium was obtained from both the wet and the dry sample.

### Example 13

Sodium mycophenolate (1 g) was dissolved at room temperature in methanol (15 ml), then diisopropyl ether (55 ml) was added to the solution. The solution was allowed to stand at room temperature overnight to promote crystallization of the product. The solid was then filtered off, and a par of the wet material was dried at normal pressure at room temperature. Form M2 of mycophenolate sodium was obtained from both the wet and the dry sample.

### Example 14

Sodium mycophenolate (1 g) was dissolved at room temperature in methanol (15 ml), then nitromethane (220 ml) was added to the solution. The solution was allowed to stand at room temperature overnight to promote crystallization of the product. The solid was then filtered off, and a part of the wet material was dried at normal pressure at room temperature. Form M2 of mycophenolate sodium was obtained from the both the wet and the dry sample.

### Example 15

Sodium mycophenolate (1g) was dissolved at room temperature in methanol (15 ml), then isobutanol (200 ml) was added to the solution. The solution was allowed to stand at room temperature overnight to promote crystallization of the product. The solid was then filtered off, and a part of the wet material was dried at normal pressure at room temperature. Form M2 of mycophenolate sodium was obtained from both the wet and the dry sample.

### Example 16

To a stirred solution of MPA (6.4 g) in dichloromethane (320 ml) and methanol (60 ml) 30% of sodium methoxide in methanol (3.8 ml) was added dropwise at room temperature. The product was precipitated by addition of n-hexane (960 ml) and filtered off. The solid material was dried at 40 °C in vacuum oven. The yield was 86%. Polymorphism was determined by X-ray diffraction. The obtained material was form M2.

### Example 17

To a stirred solution of MPA (6.4 g) in isobutyl acetate (500 ml) 30% of sodium methoxide in methanol (3.8 ml) was added dropwise at room temperature. The reaction mixture was stirred for additional 30 minutes, then the precipitated product was filtered off and washed with isobutyl acetate. The solid material was dried at 40 °C in vacuum oven. The yield was 66%. Polymorphism was determined by X-ray diffraction. The obtained material was form M2.

### Example 18

Sodium mycophenolate was dissolved at about 60°C in DMF (5 ml). The solution was allowed to cool to room temperature, then 2-propanol (300 ml) was added to the solution. The solution was stored at +4°C overnight. The solid was filtered off, and a part of the wet material was dried at normal pressure at room temperature. Forms M1+M2 of mycophenolate sodium were obtained from the wet sample. Form M2 of mycophenolate sodium was obtained from the dry sample.

### Transformation of M1+ M3 to M2 by heating

### Example 19

200 mg of a mixture of Forms M1+M3 as identified by XRD, was put into an oven in a glass weighing bottle at 170°C for 0.5 h. Then it was put into a desiccator and allowed to cool to room temperature. Upon XRD analysis, its crystal form was found to be Form M2.

### Reproduction of literature methods

### Example 20 (J. Med Chem., 39 (1996) 1236-1242)

To a stirred solution of MPA (9.6 g) in absolute ethanol (360 ml), 21% sodium ethoxide in ethanol (8.6 ml) was added dropwise at room temperature. The reaction mixture was stirred for an additional 60 minutes, then the solvent was evaporated on a rotary evaporator at 40-45°C under vacuum. The wet material was dried at 40-45°C in a vacuum oven and proved to be Form M2.

### Example 21 (ZA 68/4,959)

To a stirred solution of MPA (13 g) in chloroform (650 ml), sodium methoxide solution (2.3 g NaOMe dissolved in 130 ml of methanol) was added dropwise at room temperature. The reaction mixture was stirred for an additional 30 minutes, then n-pentane (2.34 L) was added to the solution. After 30 minutes, the reaction mixture was filtered and a part of the wet material was dried at 40-45°C in a vacuum oven. Both the wet sample and the dried material proved to be Form M2.

### Example 22 (Acta Cryst. Sect. C, C56 (2000) 432-433)

To a stirred solution of MPA (9.6 g) in methanol (300 ml), 30% sodium methoxide in methanol (5.6 ml) was added dropwise at room temperature. The reaction mixture was stirred for an additional 60 minutes, then the solvent was evaporated on a rotary evaporator at 40-45°C under vacuum. The wet material was dried at 40-45°C in a vacuum oven and proved to be a mixture of Forms M2 and M3.

Having thus described the invention with reference to particular preferred embodiments and illustrative examples, those in the art can appreciate modifications to the invention as described and illustrated that do not depart from the spirit and scope of the invention as disclosed in the specification. The Examples are set forth to aid in understanding the invention but are not intended to, and should not be construed to, limit its scope in any way. The examples do not include detailed descriptions of conventional methods. Such methods are well known to those of ordinary skill in the art and are described in numerous publications. Polymorphism in Pharmaceutical Solids, Drugs and the Pharmaceutical Sciences, Volume 95 may be used for guidance. All references mentioned herein are incorporated in their entirety.

Further aspects and features of the present invention are set out in the following numbered clauses:
1. A process for preparing anhydrous crystalline mycophenolate sodium (Form M2) characterized by a powder XRD pattern with peaks at 5.3, 8.0, 9.8, 10.7, and 21.9 ± 0.2 degrees 2-theta comprising the steps of:
   (a) preparing a solution of mycophenolic acid in a C₁-C₄ alcohol;
   (b) combining a base and a source of sodium with the solution to obtain a reaction mixture;
   (c) crystallizing the crystalline form from the mixture; and
   (d) recovering the crystalline form.
2. The process of clause 1, wherein the C₁-C₄ alcohol is methanol.
3. The process of clause 1, wherein the base or source of sodium is sodium methoxide.
4. The process of clause 1, wherein crystallization is carried out by cooling.
5. The process of clause 1, wherein step (d) is carried out after at least 5 hours of crystallization.
6. The process of clause 1, wherein the reaction is carried out at room temperature or above.
7. The process of clause 1, wherein the reaction mixture is heated to a temperature of at least about 35°C before the crystallizing step.
8. The process of clause 1, further comprising drying the crystalline form.
9. A process for preparing anhydrous crystalline mycophenolate sodium (Form M2) characterized by a powder XRD pattern with peaks at 5.3, 8.0, 9.8, 10.7, and 21.9 ± 0.2 degrees 2-theta comprising the steps of:
   (a) preparing a solution of mycophenolic acid in a C₃-C₈ ester;
   (b) combining a base and a source of sodium with the solution to precipitate the crystalline form;
   (c) recovering the crystalline form.
10. The process of clause 9, wherein the C₃-C₈ ester is ethyl acetate or isobutyl acetate.
11. The process of clause 9, wherein the base or source of sodium is sodium methoxide.
12. The process of clause 9, wherein the base is combined by adding a solution of the base in methanol.
13. A process for preparing anhydrous crystalline mycophenolate sodium (Form M2) characterized by a powder XRD pattern with peaks at 5.3, 8.0, 9.8, 10.7, and 21.9 ± 0.2 degrees 2-theta comprising the steps of
   (a) preparing a solution of sodium mycophenolate in 1-butanol;
   (b) crystallizing the crystalline form from the solution; and
   (c) recovering the crystalline form.
14. The process of clause 13, further comprising heating the solution of step (a) at a temperature of at least about 80°C.
15. The process of clause 13, further comprising heating the solution of step (a) at reflux.
16. A process for preparing anhydrous crystalline mycophenolate sodium (Form M2) characterized by a powder XRD pattern with peaks at 5.3, 8.0, 9.8, 10.7, and 21.9 ± 0.2 degrees 2-theta comprising the steps of:
   (a) preparing a solution of sodium mycophenolate in ethanol;
   (b) crystallizing the crystalline form from the solution;
   (c) recovering the crystalline form; and
   (d) drying the recovered crystalline form.
17. The process of clause 16, wherein the solution of step (a) is heated to a temperature of at least about 60°C.
18. The process of clause 16, wherein the solution of step (a) is heated to reflux.
19. The process of clause 16, wherein the ethanol is absolute ethanol.
20. The process of clause 16, wherein the recovered crystalline form is dried under atmospheric pressure at room temperature.
21. A process for preparing anhydrous crystalline mycophenolate sodium (Form M2) characterized by a powder XRD pattern with peaks at 5.3, 8.0, 9.8, 10.7, and 21.9 ± 0.2 degrees 2-theta comprising the steps of:
   (a) preparing a solution of sodium mycophenolate in ethyl lactate;
   (b) combining acetone with the solution to precipitate the crystalline form; and
   (c) recovering the crystalline form.
22. A process for preparing anhydrous crystalline mycophenolate sodium (Form M2) characterized by a powder XRD pattern with peaks at 5.3, 8.0, 9.8, 10.7, and 21.9 ± 0.2 degrees 2-theta comprising the steps of:
   (a) preparing a solution of sodium mycophenolate in dimethylformide;
   (b) combining 2-propanol with the solution to precipitate the crystalline form;
   (c) recovering the crystalline form; and
   (d) drying the recovered crystalline form.
23. The process of clause 22, wherein the recovered crystalline form is dried at room temperature.
24. The process of clause 22, wherein recovered crystalline form is dried under atmospheric pressure.
25. A process for preparing anhydrous crystalline mycophenolate sodium (Form M2) characterized by a powder XR.D pattern with peaks at 5.3, 8.0, 9.8, 10.7, and 21.9 ± 0.2 degrees 2-theta comprising the steps of:
   (a) preparing a solution of sodium mycophenolate in methanol;
   (b) combining an antisolvent with the solution to precipitate the crystalline form;
   (c) recovering the crystalline form; and
   (d) drying the recovered crystalline form,
   wherein the antisolvent is selected from the group consisting of C₃ to C₈ ketone, ester and mixtures thereof.
26. The process of clause 25, wherein the solvent is selected from the group consisting of ethyl acetate, methyl-tert-butyl ether and mixtures thereof.
27. The process of clause 25, wherein the recovered crystalline form is dried at room temperature.
28. The process of clause 25, wherein recovered crystalline form is dried under atmospheric pressure.
29. A process for preparing anhydrous crystalline mycophenolate sodium (Form M2) characterized by a powder XRD pattern with peaks at 5.3, 8.0, 9.8, 10.7, and 21.9 ± 0.2 degrees 2-theta comprising the steps of:
   (a) preparing a solution of sodium mycophenolate in methanol;
   (b) combining the solution with an antisolvent to precipitate the crystalline form; and
   (c) recovering the crystalline form,
   wherein the antisolvent is selected from the group consisting of C₃ to C₈ ketone, C₃ to C₈ ester, C₂ to C₈ ether, C₂ to C₄ alcohol, nitromethane, tetrahydrofuran and mixtures thereof.
30. The process of clause 29, wherein the solvent is selected from the group consisting of acetone, isopropanol, tetrahydrofuran, diisopropyl ether, nitromethane, isobutanol, and mixtures thereof.
31. A process for preparing anhydrous crystalline mycophenolate sodium (Form M2) characterized by a powder XRD pattern with peaks at 5.3, 8.0, 9.8, 10.7, and 21.9 ± 0.2 degrees 2-theta comprising the steps of
   (a) preparing a solution of mycophenolic acid in a mixture of dichloromethane and methanol; and
   (b) combining a base and a source of sodium with the solution to precipitate the crystalline form; and
   (c) recovering the crystalline form.
32. The process of clause 31, wherein sodium methoxide in methanol is added to the solution.
33. The process of clause 31, further comprising a C₅ to C₇ cyclic or acyclic saturated hydrocarbon before the crystalline form.
34. A process for preparing anhydrous crystalline mycophenolate sodium (Form M2) characterized by a powder XRD pattern with peaks at 5.3, 8.0, 9.8, 10.7, and 21.9 ± 0.2 degrees 2-theta comprising the steps of heating a solid mixture of a crystalline mycophenolate sodium (Form M1) characterized by a powder XRD pattern with peaks at 4.7, 6.6, 11.2 and 15.6 ± 0.2 degrees 2-theta and a crystalline mycophenolate sodium (Form M3) characterized by at least one of a powder XRD pattern with peaks at 6.0, 9.3, 15.5, and 18.4 ± 0.2 degrees 2-theta.
35. The process of clause 33, wherein the mixture of step (a) is heated to a temperature of at least about 100°C.

## Claims

1. A process for preparing anhydrous crystalline mycophenolate sodium (Form M2) **characterized by** a powder XRD pattern with peaks at 5.3, 8.0, 9.8, 10.7, and 21.9 ± 0.2 degrees 2-theta comprising the steps of:
(a) preparing a solution of mycophenolic acid in a C₃-C₈ ketone;
(b) combining a base and a source of sodium with the solution to precipitate the crystalline form; and
(c) recovering the crystalline form.

2. The process of Claim 1, wherein the C₃-C₈ ketone is acetone.

3. The process of Claim 1, wherein sodium methoxide, sodium ethoxide, or sodium hydroxide is used in step (b).

4. The process of Claim 3, wherein sodium hydroxide is used in step (b).

5. The process of Claim 1 wherein a base and a source of sodium, preferably an aqueous solution of sodium hydroxide, is added to a solution of mycophenolic acid in acetone.
